(19) **Europäisches Patentamt / European Patent Office / Office européen des brevets**

(11) **EP 2 309 253 A2**

(12) **EUROPEAN PATENT APPLICATION**

(43) Date of publication:
**13.04.2011 Bulletin 2011/15**

(51) Int Cl.:
**G01N 21/47** (2006.01)     **G01N 33/68** (2006.01)
**G01N 33/543** (2006.01)

(21) Application number: **10183562.7**

(22) Date of filing: **05.09.2002**

(84) Designated Contracting States:
**AT BE BG CH CY CZ DE DK EE ES FI FR GB GR IE IT LI LU MC NL PT SE SK TR**

(30) Priority: **05.09.2001 US 317543 P**
**12.03.2002 US 364962 P**
**24.04.2002 US 376049 P**

(62) Document number(s) of the earlier application(s) in accordance with Art. 76 EPC:
**02780278.4 / 1 432 976**

(71) Applicant: **Life Technologies Corporation Carlsbad, CA 92008 (US)**

(72) Inventors:
• **Yguerabide, Juan**
  **La Jolla, CA 92037 (US)**
• **Warden, Laurence**
  **Poway, CA 92064 (US)**
• **Bodzin, Leon J.**
  **San Diego, CA 92128 (US)**
• **Peterson, Todd**
  **Coronado, CA 92118 (US)**
• **Tenbroeck, Dirk**
  **Nashua, NH 03062 (US)**

(74) Representative: **Clarke, Jeffrey**
  **Harrison Goddard Foote**
  **40-43 Chancery Lane**
  **London WC2A 1JA (GB)**

(54) **Apparatus for reading signals generated from resonance light scattered particle labels**

(57) Embodiments of the present invention include a control and analysis system, a signal generation and detection apparatus, or reader for capturing, processing and analyzing images of samples having resonance light scattering (RLS) particle labels. An analyzer/reader includes an illumination system having a unique shutter/ aperture assembly for delivering precise patterns of light to sample, a computer controlled X-Y stage, and a detection system comprising a CCD camera to allow separation and analysis of detected light that contains information from gold and/or silver RLS labels. Alternative embodiments include linear scanning apparatus and simplified apparatus for low density samples.

*FIG. 1*

EP 2 309 253 A2

**Description**

**CROSS-REFERENCE TO RELATED APPLICATIONS**

This application claims priority to provisional U.S. applications, serial no. 60/317,543, filed on September 5th 2001, entitled "Apparatus for Analyte Assays", serial no. 60/364,962, filed March 12, 2002, entitled "Multiplexed Assays Using Resonance Light Scattering Particles," and serial no. 60/376,049, filed April 24, 2002, entitled "Signal Generation and Detection System for Analyte Assays," all of which are incorporated herein by reference in their entirety.

**FIELD OF THE INVENTION**

The present invention generally relates to apparatus for processing data obtained from assay measurements on analytes. More specifically the present invention provides apparatus for capturing, processing and analyzing images of samples having resonance light scattering (RLS) particle labels.

**BACKGROUND OF THE INVENTION**

Binding-pair techniques play an important role in many applications of biomedical analysis and are gaining importance in the fields of environmental science, veterinary medicine, pharmaceutical research, food and water quality control and the like. Such techniques rely upon an interaction, usually reversible, between a molecule of sample, and a label, or probe, that is based upon molecular recognition. The interaction may be highly specific, for example having the character of a ligand-receptor binding event, or may involve use of a substance that has very broad binding capabilities.
For the detection of analytes at low concentrations (less than about 1 picomole analyte per volume of sample analyzed), fluorescent, isotopic, luminescent, chemiluminescent, or electrochemiluminescent labels and accompanying specific detection methods are often used. Such methods are able to achieve detection of low concentrations of analytes by amplifying many-fold the number of luminescent molecules or photon-generating events. However, those methods suffer from a number of drawbacks, which makes the detection of analytes complicated, difficult, time consuming, and costly. Not least of these drawbacks are problems of interference of chemical or enzymatic reactions, contamination, complicated and multi-step work-up and analysis procedures, limited adaptability to single step homogeneous, non-separation, formats, and the requirement of costly and sophisticated instrumentation.
Recently a particularly advantageous method of detecting analytes using submicroscopic (typically nanometer-sized) metal colloidal particles as labels has been developed. The detection and/or measurement of the light-scattering properties of the particles is correlated to the presence and/or amount, or absence, of one or more analytes in a sample. Analyte detection using such a technique, and an apparatus for carrying out analyte assays, are described in, for example, Yguerabide et al., U.S. Patent No. 6,214,560, and international applications PCT/US/97/06584 (WO 97/40181), PCT/US98/23160 (WO 99/20789), and U.S. provisional patent application serial number 60/317,543 (filed September 5, 2001), each of which is incorporated by reference herein in its entirety. Elements of the basic principles behind this technology known as resonance light scattering (RLS) technology, are also described in the two publications: Yguerabide & Yguerabide, Anal. Biochem., 261:157-176, (1998); and Anal. Biochem., 262:137-156, (1998). It finds application to a wide range of situations including those where, hitherto, fluorescent labels such as fluorescein have been employed. Other investigators, for example, Schultz et al., U.S. Patent 6,180,415, Schultz et al., U.S. Patent Application Serial No. 09/740,615 and Schultz et al., Proc. Natl. Acad. Sci., 97:996-1-1 (2000) have reported on many of these properties and applications for light scattering particle labels.
RLS technology is based on physical properties of metal colloidal particles. These particles are typically nanometer-sized and, when illuminated with either coherent or polychromatic light, preferentially scatter incident radiation in a manner consistent with electromagnetic theory known as resonance light scattering. The light produced by sub-microscopic RLS particles arises when their electrons oscillate in phase with incident electromagnetic radiation. The resulting scattered light is in the visible range and is highly intense, often being at least several orders of magnitude greater than fluorescence light when compared on a per label basis. The level of intensity and color is determined largely by particle composition, size and shape.
In contrast to the use of fluorescent labels, where the analyte binds to a fluorescent molecule, or tag, whose fluorescence is detected, the principle behind RLS is that those analytes are bound to at least one detectable light scattering particle with a size smaller than the wavelength of the illuminating light. These particles are illuminated with a light beam under conditions where the light scattered by the particle can be detected. The scattered light detected under those conditions is then a measure of the presence of the one or more analytes in a sample. The method of light illumination and detection is named DLASLPD (Direct Light Angled for Scattered Light only from Particle Detected).
By ensuring appropriate illumination and maximal detection of specific scattered light, an extremely sensitive method of detection results that can enable detection of one or more analytes to very low concentrations. In fact, the light scattering power of a 60 nm gold particle is equivalent to the fluorescent light emitted from about 500,000 fluorescein molecules. It has been found that, in suspension, 60 nm gold particles can be detected by the naked eye, through observation of scattered light, at a concentration down to $10^{-15}$ M. Indeed, ultra-sensitive qualitative solid phase assays can be conducted with the naked eye, enabling detection of as little as $10^{-18}$ moles of analyte in 100 $\mu$l of analyte sample using integrated light intensity. Furthermore, single particle detection is possible by the human eye with less than 500 times magnification as viewed through an optical microscope. For example, individual gold particles can readily be seen in a student microscope with simple dark field illumination.

Additional benefits of RLS particles include the fact that they do not photobleach, the color of the scattered light can be changed by altering particle composition or particle size, and the particles can be coated with antibodies or DNA probes for detection of specific analyte antigens or DNA sequences. Furthermore, RLS particles offer a broad dynamic range: by judicious choice of integrated light intensity measurements or direct observation by eye, analyte can be detected over three decades of analyte concentration, and the region of dynamic range can be adjusted by changing the particle size. RLS particles are also compatible with homogeneous assays, for example in solution, or in solid phase assays wherein very high sensitivity can be obtained through particle counting. In short ultra-sensitive quantitative assays can be conducted with relatively simple instrumentation.

Nevertheless, RLS particles cannot be detected with conventional laser readers due to the fact that the particles emit the same wavelength light as the excitation source. Laser readers are based on the Stokes shift phenomenon of fluorescence, in which the emitted light from a fluorescent molecule is of a longer wavelength than that of the excitation light. Laser readers are designed to block the excitation spectrum from the detection system, allowing only the emission from the fluorescent molecule to be sensed by the detector. Therefore, because existing laser based systems are tailored to fluorescence labeling, specific instrumentation has been developed to analyze microarrays labeled with RLS particles.

In essence, RLS technology can detect low concentrations of analytes without the need for signal or analyte molecule amplification. Furthermore, the method provides a simplified procedure for the detection of analytes wherein the amount and types of reagents are reduced relative to other methods in the art. The method also enables substantial reductions in the number of different tests, thereby leading to reduced costs and lower production of waste, especially medically-related waste that must be disposed of by specialized means. The method has found application to techniques including, but not limited to: *in vitro* immunoassays; *in vitro* DNA probe assays; gene expression microarrays; DNA sequencing microarrays; protein microarrays; immunocytology; immunohistochemistry; *in situ* hybridization; multiplexed (multicolor) assays; homogeneous immuno, and DNA probe assays; and microfluidic, immuno, and DNA probe assay systems.

The wide range of specific light scattering signals from different particle types means that one skilled in the art can detect and measure to a high degree of specificity one or more analytes in a sample. Where there is high optical resolvability of two or more different particle types there is the potential for very simple multi-analyte detection (*i.e.*, simultaneous detection of two or more different analytes) in a sample without the need for complex apparatus. Furthermore, the use of specific particle types that possess highly measurable and detectable light scattering properties in a defined assay format enables ready application of the method to micro-array and other high-throughput techniques.

A particularly important area for application of microarrays is gene expression analysis. A basic problem in expression analysis is the determination of gene regulation profiles while compensating for unassociated assay and system variations. Gene regulation profiles are used to determine the degree to which a particular sample of genetic material is expressed relative to another, under controlled experimental conditions. However, independent assay and system variations can act to obstruct accuracy in such comparative expression studies.

Sources of variation are experiment dependent. A list of commonly contributing factors from Schuchhardt, J., Beule, D., Malik, A., et al., "Normalization Strategies for cDNA Microarrays," Nucl. Acids Res., 28: e47, (2000) is included herein below . This list of factors addresses fluctuations in probe, target and array preparation, in the hybridization process, background and overshining effects and effects resulting from image processing, but is not intended to be an exclusive list.

Modest increases in gold particle size results in a large increase in the light scattering power of the particle (the $C_{sca}$). The incident wavelength for the maximum $C_{sca}$ is increased significantly with particle size and the magnitude of scattered light intensity is significantly increased. This further shows that when illuminated with white light, certain metal-like particles of identical composition but different size can be distinguished from one another in the same sample by the color of the scattered light. The relative magnitude of the scattered light intensity can be measured and used together with the color or wavelength dependence of the scattered light to detect different particles in the same sample more specifically and sensitively, even in samples with high non-specific light backgrounds.

Thus there remains a need for a flexible, highly sensitive and automated signal generation and detection system capable of capturing, processing and analyzing various formats of samples having RLS particle labels formats. The commercial availability of such a system and method would have wide applicability and benefit research and industry in many applications including microarrays, biochips, genomics, proteomics, combinatorial chemistry and high throughput screening (HTS).

## SUMMARY OF THE INVENTION

The present invention provides an ultra-sensitive signal generation and detection system for multiplexed assays of analytes. The system enables simple and efficient detection using Resonance Light Scattering (RLS) particles and a signal generation and detection apparatus to facilitate the measurement and analysis of biological interactions on a variety of solid phase formats including glass, plastic and membrane substrates and microwell plates. Certain embodiments are designed for use with arrays, and are particularly advantageous for microarrays, in view of the high feature density and large amounts of data potentially generated from even a single microarray.

The present invention is based on physical properties of submicroscopic (nanometer-sized) metal colloidal particles. These particles, when illuminated with either coherent or polychromatic light, preferentially scatter incident radiation in a manner consistent with electromagnetic theory known as resonance light scattering (RLS). The light produced by sub-microscopic RLS Particles arises when their electrons vibrate/oscillate in phase with incident electromagnetic radiation. The resulting signals are in the visible range and are highly intense, often being at least several orders of magnitude greater than fluorescence on a per label basis. The level of intensity and color is determined by particle composition, size and shape.

A preferred embodiment of the signal generation and detection system of the present invention includes a control and analysis system, a signal generation and detection apparatus, or reader, and companion software for controlling the reader and for capturing, processing and analyzing RLS images and other data. The reader includes an illumination system having a unique shutter/aperture assembly for delivering precise patterns of light to a sample, a computer controlled X-Y stage, and a detection system comprising a CCD camera. The system may be operated manually or via software instructions and algorithms for generating, capturing, processing and analyzing RLS images. For example, the control system performs multiplexed assays of two or more colors, e.g., to allow separation and analysis of detected light that contains information from nanometer gold and silver RLS labels.

In alternative embodiments of the invention, a fluid filled imaging chamber is provided to reduce light scattering and optimize imaging. Similarly, linear light imaging may be employed with a linear lens and scanning movement of the sample holder. In another embodiment, low cost photomultipliers or photodiodes are used as detectors for low density samples.

## BRIEF DESCRIPTION OF THE DRAWINGS

These and other features, aspects, and advantages of the present invention will become more readily apparent from the following detailed description, which should be read in conjunction with the accompanying drawings in which:

FIG. 1 is a perspective view of a signal generation and detection system for analyte assays according to the present invention.
FIG. 2 is a schematic top view of a reader apparatus of the signal generation and detection system of FIG. 1, with the cover removed;
FIG. 2A is a schematic side view of the reader apparatus of FIG. 2;
FIG. 3 is a cross-sectional schematic view of the illumination system of the reader apparatus of FIG. 2;
FIG. 4A is a perspective view of the shutter/aperture assembly of the illumination system of FIG. 3;
FIG. 4B is a cross-sectional view of the shutter/aperture assembly of FIG. 4A;
FIG. 4C is a perspective view of the rotary drum of the shutter assembly of FIG. 4A;
FIG. 5 is a top view of the reader apparatus of FIG. 2, showing the multi-format substrate holder with a micro-well plate;
FIG. 6 is a schematic section diagram illustrating an LED ring illuminator and associated housing as used with a low cost RLS-based analyzer;
FIG. 7 is a schematic diagram of a hexagonal immersion tank for suitable for RLS detection;
FIG. 8 is a schematic diagram of a low volume immersion tank suitable for RLS detection;
FIG. 9 is a schematic diagram of an alternative reader apparatus with a linear illumination and detection; and
FIG. 10 is a schematic diagram of optics of an alternative reader apparatus having a photodiode or photomultiplier detector.

## DETAILED DESCRIPTION OF THE INVENTION

The structure and function of the preferred embodiments of the apparatus and methods of the present invention can best be understood by reference to the drawings. Where the same reference designations appear in multiple locations in the drawings, the numerals refer to the same or corresponding structure in those locations. While the invention will be described in conjunction with the preferred embodiments, it will be understood that they are not intended to limit the invention to those embodiments. On the contrary, the invention is intended to cover alternatives, modifications, and equivalents, which may be included within the invention as defined by the appended claims. Before addressing details of the invention, the following definitions are provided. These definitions are provided solely for the convenience of the reader and are not to be considered an exhaustive list of terms used in describing the various embodiments of the present invention.

By "light" is meant ultraviolet, visible, near infrared, infrared, and microwave frequencies of electromagnetic radiation.

By "analyte" is meant material evaluated in an assay. The analyte is typically just one of a complex mixture of materials but is a specific material of interest to a researcher, technician or other professional person. Such material is preferably an organic substance and is preferably detected indirectly through detection of a particle or label to which it binds. If the analyte is detected through its interaction with a particle, then the particle preferably has immobilized thereon a compound or molecule that is potentially capable of binding with the analyte. In some embodiments, an interaction between the analyte and another species is indirectly analyzed with a reporter species that specifically detects the interaction. For example, binding between an immobilized antigen and a first antibody (or visa versa) could be analyzed with a labeled second antibody specific for the antigen-first antibody complex. For applications involving hybridization between polynucleotides, the presence of hybrids could be detected by intercalating dyes, such as ethidium bromide, which are specific for double-stranded polynucleotides. In such situations, it is the reporter species that is detected and correlated with presence of analyte.

An assay may be able to simultaneously detect the presence of more than one analyte of interest in a sample. An assay may also be able to identify multiple compounds which interact with an analyte of interest, such as, for example, to identify a peptide or other compound which binds an antibody, enzyme or other receptor of interest. When the immobilized compounds on particles are polynucleotides, the assays are particularly advantageous for use in hybridization-based applications such as sequencing. Furthermore, arrays suitable for use with the present invention are not limited to applications in which an immobilized compound and analyte bind another. The arrays can also be used to screen for and identify compounds which catalyze chemical reactions, such as antibodies capable of catalyzing certain chemistries, and to screen for and identify compounds which give rise to detectable biological signals, such as compounds which bind to a receptor of interest. The only requirement is that the interaction between the immobilized compound and an analyte give rise to a spatially-addressable detectable signal. Thus, the present invention is useful in any applications that take advantage of arrays or libraries of immobilized compounds, such as the myriad solid-phase combinatorial library assay methodologies described in the art.

The term "light scattering particles" refers to particles that scatter light of visible wavelengths sufficiently strongly to be useful as labels in analyte assays. For example, such particles include metal or metal-like materials as described herein. It is recognized that all particles will scatter light to some extent.

As used herein, the term "array" refers to a plurality of sites in or on a single physical medium (also referred to as sample format or assay format), *e.g.*, a slide, chip, or membrane. Preferably the sample format is a solid phase material with a substantially flat sample-bearing or sample-binding surface. Preferably, an array includes at least 50 separate sites. In alternate embodiments, an array can include 6, 8, 10, 20, 100, 200, 400, 800, or 1,000, 5,000, or 10,000 separate sites.

The term "assay format" refers to the physical medium or physical layout of the assay component in, or on which, label is detected and/or from which label is released for detection. The assay format may also be referred to as sample format. Different assay formats include, for example, slide, chip, membrane, microtiter plate such as a 96-well plate, flow cell, cuvette, and gel (*e.g.*, polyacrylamide or agarose gel) formats. The particles used in conjunction with the present invention are compatible with both solution and solid phase assays. In solution, the particles form a very fine suspension.

The terms "spots" and "features" are also used to refer to sites. In connection with multiple site assay formats, the term spot will be used to refer to a site (*i.e.*, a spot) on an array. Often the term feature will be used in this manner, but can also be used to refer to other formats.

As used herein, the terms integrated intensity, integrated light, integrated signal and like terms refer to the light collected over an area of interest for a period of time, rather than to the general collection of instantaneous light intensity. Thus, the amount of light collected will depend on the area of interest, the collection period, the average light intensity over that period, and the collection efficiency of the collector or sensor.

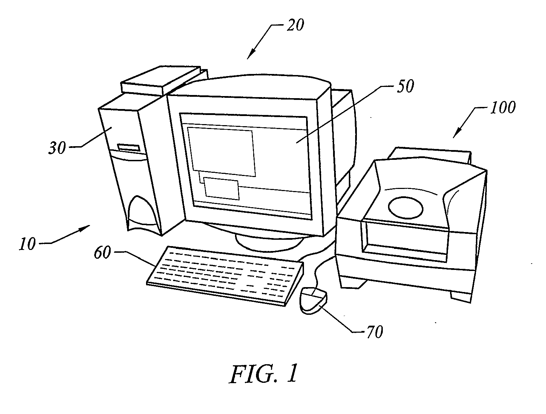

Referring now to FIG. 1, a preferred embodiment of a RLS signal generation and detection system 10 according to the present invention comprises control and analysis system 20 and signal generation and detection apparatus (also referred to as the reader) 100. Control and analysis system 20 generally includes at least one computing device 30 and a user interface, which in turn comprises a display 50 and one or more user input devices such as a keyboard 60, mouse 70 and/or other devices for inputting information or commands. Preferably, the system will provide for visual inspection of the sample signal, e.g., via a microscope ocular lens (not shown) and/or via display 50.

In an exemplary embodiment, computing device 30 is a computer system with a central processing unit (CPU) *e.g.*, such as a 1 GHz or faster Intel Pentium processor, volatile memory, non-volatile memory, a removable media drive (such as a CD-RW drive), and an interface bus for communication with the user interface and the scanning apparatus. One of ordinary skill in the art will appreciate that control and analysis system 20 may be any computer or other processor-based system with sufficient resources to store and implement software instructions and algorithms to control signal generation and detection apparatus 100 and to capture, process, and analyze RLS images as desired for a given application.

In an exemplary instrument, reader control software controls all the functions of the system including the stage, camera, filters, image correction and instrument setup and maintenance routines. However, it is apparent that one or more of these processes may be done manually, or with partial manual control and/or evaluation. Individuals familiar with automated instrumentation are familiar with such software and associated routines, and can readily select or write suitable software or component routines for a particular instrument design. Such reader control software may include routines and algorithms for calibrating and/or correcting images, such as bias frame correction, flatfield correction, and instrument normalization.

The apparatus of the present invention are advantageously applied to detection and measurement of one or more analytes in a sample, especially to analyte detection and/or quantitation methods based on the use of types of particles of specific composition, size, and shape (RLS particles), and the detection and/or measurement of one or more light scattering properties of the particles. Certain embodiments are designed for use with arrays, and are particularly advantageous for microarrays, in view of the high feature density and large amounts of data potentially generated from even a single microarray.

In typical assays, one or more types of metal-like particles are detected in a sample by measuring their color under white light or similar broad band illumination with illumination and detection methods as described herein or in U.S. provisional application serial nos. 60/317,543 and 60/376,049. For example, roughly spherical particles of gold (which may be coated with binding agent, bound to analyte, released into solution or bound to a solid-phase) of 40, 60, and 80 nm diameters, and a particle of silver of about 60 nm diameter, can easily be detected and quantified in a sample by identifying each particle type by measuring the unique color and/or the intensity of their respective scattered light. This can be carried out on a solid phase such as a microtiter well or microarray chip, or in solution. The measurement in solution is more involved, because the particles are not spatially resolved as in the solid-phase format. For example, one can detect the different types of particles in solution by flowing the solution past a series of detectors, each of which is set to measure a different wavelength or color region of the spectrum and its respective intensity. Alternatively, a series of different wavelengths of illumination and/or detection can be used with or without the flow system to detect the different particle types.

For solid-phase analytical applications, a very wide range of concentrations of metal-like particles is detectable by switching from particle counting to integrated light intensity measurements, depending on the concentration of particles. The particles can be detected from very low to very high particle densities per unit area. This can be accomplished by fitting the instrument with a dual magnification lens system which can initially image the sample at low magnification to gain integrated intensities of areas of interest, then re-image selected areas under high magnification, with the necessary spatial resolution necessary for particle counting.

In other assay applications, the particles which are bound to a solid substrate such as a bead, solid surface such as the bottom of a well, or other comparable apparatus, can be released into solution by adjusting the pH, ionic strength, or other liquid property. Higher refractive index liquids can be added, and the particle light scattering properties are measured in solution. Similarly, particles in solution can be concentrated by various means into a small volume or area prior to measuring the light scattering properties. Again, higher refractive index liquids can be added prior to the measurement.

Additional description and details of an exemplary system according to the present invention are included in the user manual for the Genicon Sciences, Inc. GSD 501 system, entitled "GSD-501 System: RLS Detection and Imaging Instrument," available at www.genicoisciences.com/root/files/1280.pdf, in its entirety.

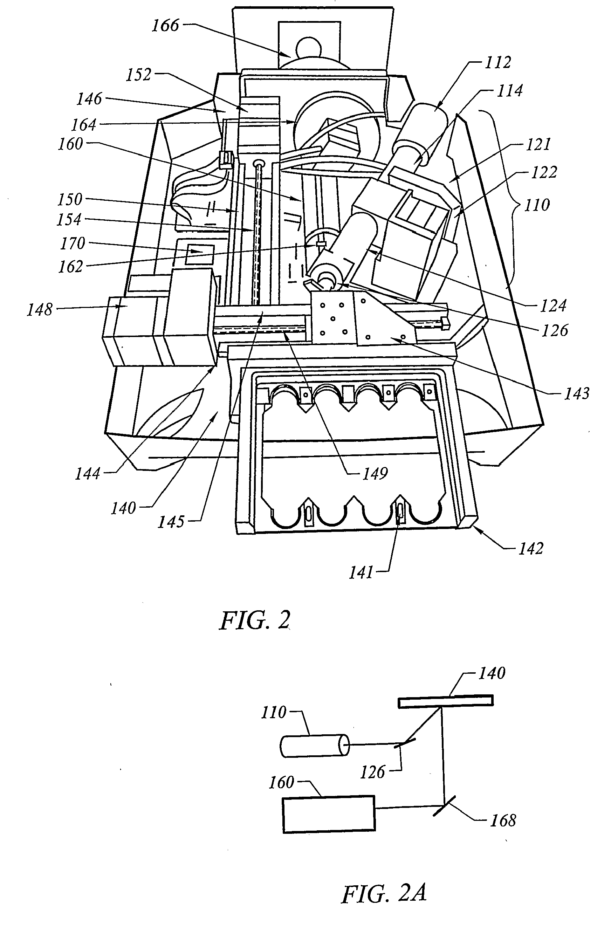

As shown in FIG. 2, reader 100 according to one preferred embodiment comprises illumination system 110, multiformat sample holder subassembly 140, detection system 160 and control electronics 170. Reader 100 may also include communications ports such as an RS232 or Ethernet port and a data port for communication between control electronics 170 and control system 20. A power connection provides power (e.g. 110-240VAC, 47-63 Hz and 3 amps) to the reader power supply (not shown). Any suitable power supply may be used, however a preferred embodiment uses a power supply having an output of 24VDC and 4 amps. Typical locations for such ports/connections are on the back panel of the device. A fan and filtered air inlet also may be provided on the back panel to cool the device..

Illumination system 110 responds to commands from control electronics 170 to illuminate a discrete area of a slide or other sample substrate held by sub-assembly 140, such that RLS particles in the illuminated area may be detected by detection system 160. Control electronics 170 communicates with control and analysis system 20 in order to provide appropriate illumination for the sample being examined. In a preferred embodiment, illumination system 110 is disposed above detection system 160, as shown in FIG. 2A. Using mirror 126, light from the illumination system is directed upward at an angle of approximately 25 degrees to an illumination and detection area on multiformat sample holder subassembly 140. Scattered light from detected particles is directed downward and reflects off mirror 168, preferably at about 45 degrees, into detection system 160.

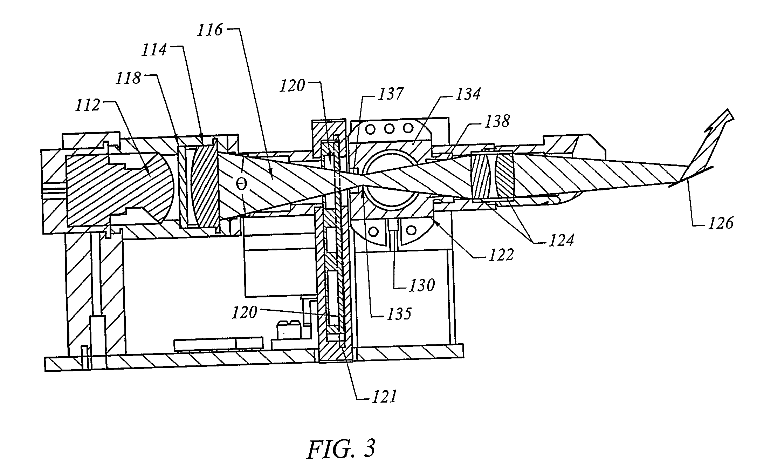

As shown in greater detail in FIG. 3, illumination source 112 of illumination system 110 provides light that passes through focusing lens 114 to form light cone 116. Lens 114 may be configured to provide light cone 116 at a particular angle θ, for example about 20.6 degrees, or at a sufficient angle required to fully illuminate the aperture located in the shutter. Depending upon the particular application, light cone 116 optionally passes through an infrared cutoff filter 118 and/or an optical band pass filter 120 before passing through an aperture in shutter/aperture assembly 122. The infra red filter may be used to reduce heating of the sample due to the projected light. Optical band pass filter 120 is selectable, supported on filter wheel 121. The optical band pass filter is used to select appropriate wavelengths of light for the sample and format to be analyzed. For example, the wheel may support filters of 450 x 70, 565 x 70 and 600 x 70 nanometers. Imaging lens set 124 focuses the light onto illumination mirror 126 or other light-guiding system, which directs the focused light to illuminate the sample.

In light scattering configurations, light source 112 may comprise an arc lamp, such as a 10W metal halide arc lamp. In alternative embodiments, the light can be polychromatic or monochromatic, steady-state or pulsed, and coherent or non-coherent light. It can be polarized on unpolarized, and can be generated from a low power light source such as a filament bulb, laser or a light emitting diode (LED), depending on the desired incident wavelength range. Alternatively, light may be delivered using a fiberoptic apparatus or a liquid light guide. In either configuration, the sample is illuminated by either epi- or trans- means in a dark field configuration. The illumination is introduced to the sample in a manner and at angles to avoid creating reflections of incident light into the detection optics, e.g., from either the sample surface, or from any other reflective surface after the sample. In a preferred embodiment, illumination system 110 is automatically controlled to precisely deliver a specific light pattern to a particular area of the sample for a desired exposure.

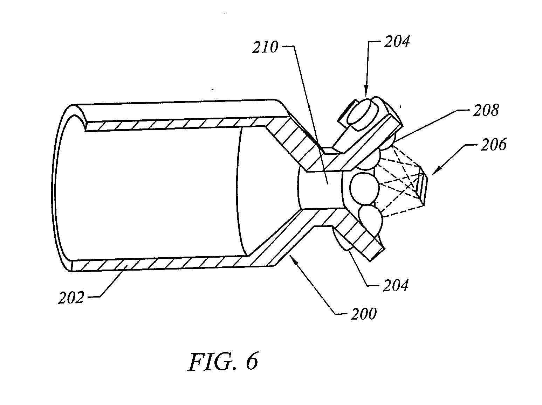

In a further alternative embodiment, an annular light source may be implemented as illumination source 112. By way of example, such a light source may comprise a plurality of LEDs arranged in a ring (see, for example FIG. 6). Alternatively, the annular ring light may include a ring-shaped LED. When using plural LEDs, they may be combined at different intensities and wavelengths to create a tunable light source to provide illumination of a specific wavelength at the targeted area of illumination and thereby obviate the need for one or more filters. The output intensity of the LEDs can be controlled in at least two ways, either by switching the number of LEDs that are on, and/or by regulating the output from the individual LEDs. The output can be regulated in various ways. For example, the output can be controlled by a resistor network connected to a rotary switch or potentiometer. Alternatively, the intensity can be controlled via computer. Such output control circuits and/or computer control software and hardware are well-known and can be readily adapted to the present implementation. Rather than a single ring of LEDs, one or more additional rings can be used, for example provided on a wheel and selectable thereby. The multiple rings can be used separately or together to provide intensity control and/or color control.

Intensity control is particularly useful to provide signal optimization. For example, an illumination intensity or intensities can be selected to provide convenient signal detection without saturating the detector sensor (e.g., camera). While such intensity levels can be established using visual feedback, preferably the feedback and intensity setting is performed using computer control. In this process, the signal intensity is read, and the illumination intensity adjusted as needed to increase or decrease the signal intensity, or to leave it unchanged.

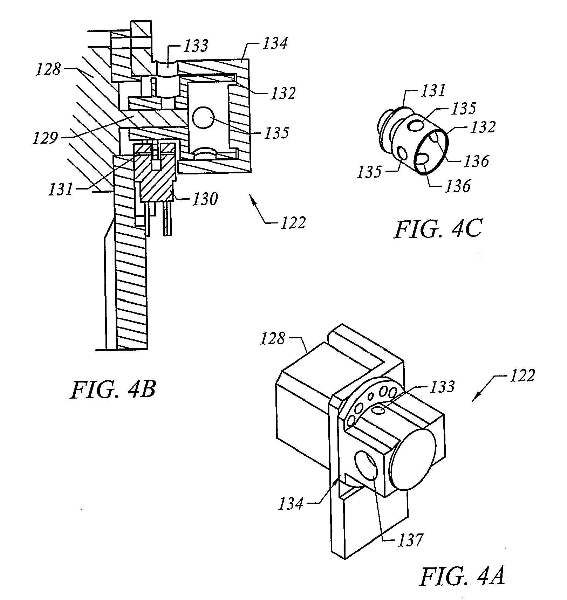

One means for specifically delivering desired light patterns to selected areas of the sample is shutter /aperture assembly 122. Shutter/aperture assembly 122 also controls the length of exposure. As shown in FIG. 3 and FIGS. 4A-4C, in a preferred embodiment of the present invention, assembly 122 uses high-speed stepper motor 128 and encoder 130 to precisely position rotary drum 132 for both presenting the necessary aperture to the delivery optics and for shuttering the light. Rotary drum 132 is mounted on motor shaft 129 and includes encoder wheel portion 131 cooperating with encoder 130. In an exemplary embodiment, a set screw may be placed through opening 133 in housing 134 in order to secure the drum on the motor shaft. Opposite the motor shaft, rotary drum 132 defmes several apertures 135, such as thin photo-etched apertures, located radially around the circumference. Each aperture may present a different shape and size, permitting precise sections and control of the illumination area of the sample. Opposite each aperture 135, the drum defines larger clearance holes 136. The area between the apertures is filled with opaque sections of the drum, which are capable of blocking the light. Openings 137 and 138 in housing 134 permit the light to pass through.

In use, the light from illumination source 112 is directed at the drum, perpendicular to the axis of motor shaft 129. Under control of control electronics 170 (and, alternatively, also control system 20), motor 128 rotates drum 132 such that an aperture and its corresponding clearance hold is located in line with source 112 and the pattern of the aperture is projected onto the sample. Preferably, this rotation happens very rapidly, e.g., in a matter of milliseconds. Once the desired exposure has been reached, the motor shaft 129 rotates, preferably in the same direction, such that a solid area of drum 132 blocks the light. The process is repeated on the next exposure with the motor running in the reverse direction. The effect of rotating apertures 135 through the exposure in the same direction causes a first-in, first-out effect on the illumination pattern, resulting in more even illumination at short exposures. The precision of the movement optimizes system performance and is controlled by encoder 130. Preferably, the system is capable of exposures of 0.040 seconds or less. In an alternative embodiment, two distinct assemblies may replace the shutter/aperture assembly, e.g., a multiple aperture assembly controlled by a computer and a separate shutter device, such as a vane shutter, to intermittently block and permit the passage of light.

Slides or other sample carrying substrates are moved over the fixed illumination and detection area by multi-format sample holder subassembly 140. Substrate holder 142 is provided as an open structure to carry a variety of different sample containing substrates. Spring clips or detents 141 may be provided to help hold the different substrates in place. As shown in FIG. 2, substrate holder is configured for holding slides of example only. As will be recognized by person of ordinary skill in the art, and as explained in more detail below, other types of substrate holders may be used without departing from the scope of the invention.

High-precision XY stages144, 146 provide the movement that allows capture of individual images at precise locations on the sample. Substrate holder 142 is mounted on carriage 143, which is carried by X stage 144. Carriage 143 is preferably designed so that different substrate holders may be easily attached and removed. X stage 144 includes rails 145 on which carriage 143 rides. An encoder controlled, stepper motor 148 drives power screw 149 that positions the carriage and substrate holder. X stage 144 is mounted on Y stage 146 and carried on Y stage rails 150. Another encoder controlled, stepper motor 152 drives power screw 154 for positioning X stage 144 in the Y direction. Both stepper motors operate under control of the control electronics 170 and the over all control system 20. Exemplary XY stages include, for example, stages from Conix Research, e.g., the Conix Stages 6400RP and 4400LS stages, the OptiScan stages from Prior Scientific, Inc., and stages from Applied Scientific Instrumentation, Inc., among others.

Calibration of the movement of the X-Y stages 144, 146 and the magnification of the optical system may accomplished through the use of a specially designed photo-lithographed calibration slide. Such a slide has features designed to aid in analyzing the distance between imaging areas and the orientation of the detection system 160 to the stages. The stage calibration slide may be used in conjunction with an automated software routine, for example imbedded in reader control software, to tune the stage positioning without technician support.

Referring again to FIG. 2, detection system 160 preferably includes detection optics 162, detection filter wheel 164 and electronic sensor 166. Electronic sensor 166 can be of various designs, for example, CCD, CMOS, and CID sensors, typically in a camera. The sensor can be an averaging sensor or an imaging sensor. In lower cost systems, preferably a CCD or CMOS camera would be used. The sensor may be an anti-blooming system, e.g., using lateral overflow drains for CCD sensors. In a preferred embodiment, sensor is a cooled scientific grade CCD camera.

The sensor or camera output can be directly fed to a visualization system, but is preferably directed to control and analysis system 20 to analyze the signal to identify and/or quantitate signal in areas of interest. In either case, the image and/or other signal characteristics can be stored electronically and/or on hard copy. Such storage is well known for various image data, and includes film, printer output, and the various computer electronic storage media, e.g., disk, tape, CD-ROMs, etc.

Depending on the resolution required, for example at 5 micron resolution, capturing the entire slide in one image would require a CCD sensor with over 62 million pixels. Massive memory and computing power would also be required to process such an image. However, CCD arrays (or other types of arrays) are generally not available in such large size, and such computer requirements are currently impractical and expensive. Thus, in a preferred embodiment of the invention, smaller CCDs are used to image multiple tiles across the slide, which are then assembled together using image processing software on the host computer 30. In order to perform this sub-image assembly, the sample is moved in precise increments in X and Y directions, as permitted by the multiformat sample holder sub-assembly 140, as described above. Without such precise movements, image artifacts may be introduced, or substantial additional image analysis may be needed to correctly align adjacent image tiles. This movement is accomplished by the precision computer controlled XY stages 144, 146 with an accuracy and repeatability of preferably less than 3 $\mu$.

In order to accurately assemble the sub-images into a single composite image, a determination of the spatial relationship between the motion of the X-Y stage to detected positional shift(s) of the sample must be established. This can be accomplished by at least two approaches. In one approach, a patterned image target is placed in the image plane with accurately positioned landmarks that can be clearly detected by the detection system. Images of the target are analyzed to determine the spatial relationship between the landmarks and the number of pixels in the image. From this relationship, the necessary stage motion in both the X and Y axes are used to index the sample one complete frame.

In another preferred approach, the patterned target is built into the X-Y stage of substrate holder 142, coplaner with the sample measurement surface. The patterned target contains at least one feature, such as a contrasting, small filled circle that is easily detectable. Am automated software routine may be used to capture an image of the target at a specific location. The X-Y stage is then moved so as to position the target in different areas where different images are captured. The images are subsequently analyzed to determine the relationship between the distances the stage has been commanded to move versus the distance in pixels the image has detected. Since the number of pixels of the imager frame is fixed, the spatial relationship between the stage motion and the image frame can be determined. In order to improve precision, the process may be repeated over several positions across the image frame using a statistical treatment, for example by averaging. A person of ordinary skill in the art may devise suitable software routines based on the teachings provided herein.

Detection optics 162 may include a fixed magnification lens, preferably a 2 or 4X lens, or more preferably a multi-position computer controlled zoom lens, allowing the operator to choose the magnification required for the experiment. With a fixed magnification system, the optical resolution is defined by the size of each pixel and the magnification power of the lens. This is defined as the base magnification, usually 4X. If the user desires to select a lower magnification to reduce the image file size, the system will typically perform pixel binning, whereby the signal of two or more pixels are summed together to effectively increase the size of each pixel, producing an image at lower resolution.

The disadvantage of this approach is that the camera still has the same field of view even though the resolution is lower, resulting in the same number of images to cover the slide as the high-resolution setting. If the lens magnification of the system can be changed instead of simply binning pixels, for example from 4X to 2X, the field of view increases, requiring fewer images to cover the slide. Acquiring fewer images reduces the time required to complete a scan.

For simplicity sake, the current exemplary system design is fitted with two, fixed zoom settings at 2 and 4X. However, an infinite level of zoom can be provided with computer control and encoder feedback. Ultimately, the magnification could be as great as 40 or 60X, allowing individual particle counting to be performed on individual spots and increasing the dynamic range of the system. While the design of the instrument preferably utilizes an infinitely adjustable para-focal zoom lens, a fixed lens system could also be utilized with two or more mechanically switched lenses in the optical path controlling the final system magnification. Detection optics 162 also preferably include an autofocus attachment, and when coupled with the appropriate software routine, can adjust the focus for differences in the distance between the camera lens and the sample to better address potential manufacturing variations in the sample substrate.

The exemplary analyzer/reader 100 also may be fitted with a second filter wheel 164 on the emission side between detection optics 162 and the camera 166. This second filter set allows the system to measure fluorescence in addition to RLS particles for controls or other applications. The excitation and emission filter wheels can be independent units or combined into one larger wheel which has opposing emission and detection filters positioned in the path of illumination and detection.

In particular embodiments, two or more different wavelengths either from the same light source or from two or more different light sources are used to illuminate the sample, and the scattered light signals are detected. The different wavelengths are provided by passing the light through band pass filter 120. Alternatively, optical filtering can be performed on the detection side, for example by filter wheel 164 of FIG. 2. In a preferred approach, the illumination is filtered with a 10, 20, 30 or 40nm bandpass filter centered about the peak scattering wavelength of the particle being measured. In an exemplary configuration, a computer controlled filter wheel is place in the light path, which can accommodate a number of filters for different sized or composition particles. For imaging samples having two different types of RLS particles, for example, two or more filters may be used to determine the relative contributions of each particle type.

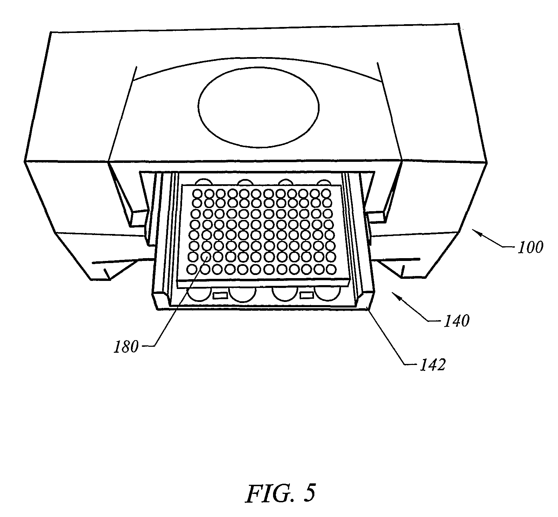

As mentioned above, substrate holder 142 in FIG. 2 may hold a number of alternative sample presentation substrates. In a preferred embodiment, reader apparatus 100 is particularly applicable to DNA and protein microarrays spotted on a substantially 1 X 3 inch glass or plastic microscope slides, patterned in 96-well Microtiter plates or spotted on immobilized membranes. Substrate holder 142 is thus designed with a removable insert, which can accommodate different holders for the desired substrate in a particular application. For example, a slide is illustrated in FIG. 2, designed to accommodate up to 4, 1 X 3 inch slides 270 at a time. Another alternative is shown in FIG. 5. Microtiter plate holder 180 one or more microtiter plates, such 96 or 384 well clear bottom microtiter plates. In other embodiments, substrate holder 142 accommodates a number of membrane carrier plates (not shown) providing appropriate areas to fit within the holder. Thus, for example, the membrane holder may be dimensioned to accommodate 1, 2, 4, 6, 8 or more membrane carriers, as well as other numbers of carriers. Provision also may be made for applying a clarifying solution between the membrane carrier plates. One skilled in the art will appreciate that multiformat substrate holder 142 may be configured to hold any other number, type, or size of samples or sample substrates of convention or custom design. Alternative sample containers or substrates include test tubes, capillary tubes, flow cells, microchannel devices, cuvettes, dipsticks, or other containers for holding liquid or solid phase samples. In a preferred approach, design of the sample holder allows easy removal and insertion using other automation components, allowing for integration into larger sample processing systems.

It will be appreciated by those skilled in the art that the novel combination of the multiformat sample holder sub-assembly and the selectable rotating aperture drum provides a unique capability to analyze samples of different formats, e.g. slides, microtiter plates and membranes or others, a single instrument. For example, spots on slides or membranes may require a square illumination area of a particular size, whereas samples in microtiter plates may require square or circular illumination of different sizes. When using different microtiter plates, the illumination area must be specifically sized to correspond to the size of the well, i.e. preferably slightly smaller than the well. The rotating drum aperture allows the different illumination requirements to be meet easily and quickly, simply by rotating the drum to a different aperture as described above. Thus, experiments may be efficiently switched between format types, with the same piece of equipment. In addition, the versatility of the instrument is further enhanced by the ability to quickly and easily change the aperture drum, thus further extending the number of usable formats.

Those of skill in the art will also appreciate that the multiformat aspects of the present invention are not limited to application with the specific light scattering particle detection described above. For example, another technique for illumination involves the use of substantially planar substrates including at least one configured diffraction or optical grating. When incident light is provided at a specific angle matching the grating, the light is coupled to the planar substrate to generate an evanescent field on or about the substrate surface on which labels associated with specific analytes are excited or illuminated for detection. Examples of such techniques are described in U.S. Patent Nos. 6,395,558 and 5,599,668, which are incorporated by reference in their entirety. Also, in addition to light scattering detection, other detection systems, such as fluorescent, luminescent, or chemiluminescent, electrochemiluminescent may be designed to accommodate multiformat sample presentation by a person of ordinary skill in the art based on the teachings of the present invention.

In another embodiment of a device for RLS detection, a light source is arranged in a ring close to the sample, as in FIG. 6, with the light output directed so as to provide dark field illumination. More specifically, annular light source 200 may comprise housing 202 supporting several white or colored LEDs 204. Housing 202 is adapted to be placed over the objective lens of a viewing or detecting device, such as a microscope or CCD camera. The dark field image of the scattered light is then viewed at target area 206 through the housing. LEDs 204 may be combined at different wavelengths and intensities to create a tunable light source to provide illumination of a specific wavelength at the targeted area of illumination 206 on the sample surface. Each LED is preferably associated with a lens or lenses 208 to control the focus and direction of the light emitted. The LEDs are focused on the field of view for a scattered light detector.

In general, LEDs in such embodiments can also be positioned so that the illuminating light passes through apertures, thereby controlling stray light that could otherwise be picked up by a detector. The size, position, and shape of the apertures is selected to allow illumination of only the desired spot or area of interest. The position and/or shape of the apertures can be controlled, preferably via computer, for example, as described above. Rather than a single ring of LEDs, one or more additional rings can be used. Preferably all the LEDs are directed to illuminate the same spot. The multiple rings can be used separately or together to provide intensity control and/or color control. Intensity and wave length control may be accomplished as previously described in connection with alternatives for light source 112.

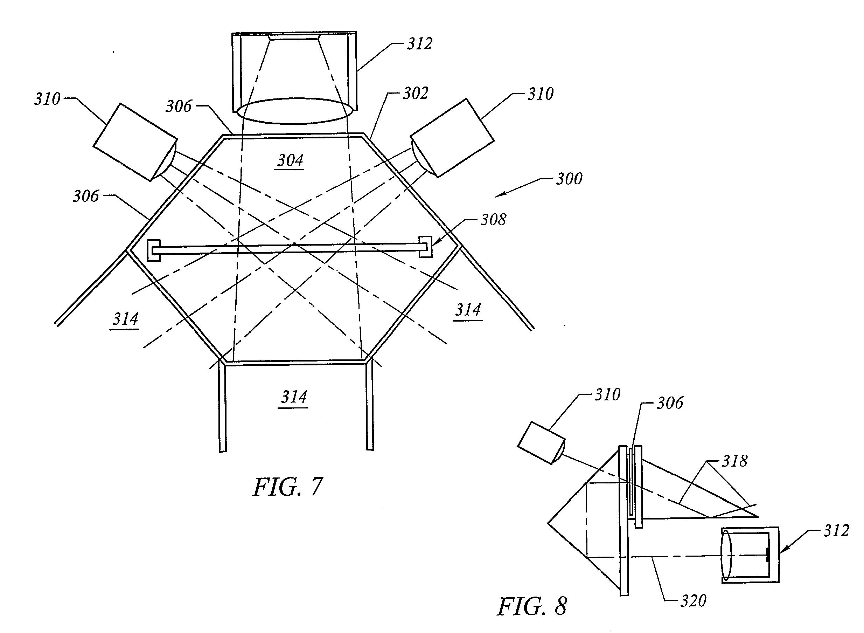

In another aspect of the invention, an immersion tank is provided for RLS detection. As shown in FIG. 7, system 300 includes an immersion tank 302 that defines a liquid filled sample device chamber 304. The chamber includes a plurality of adjoining surfaces 306, wherein at least one of those surfaces is an optically transmissive surface having a refractive index matching liquid in chamber 304. Sample holding device 308, including a sample with light scattering particle labels bound thereto, is disposed in chamber 304. The sample device is immersed in the liquid and the light scattering labels are illuminated by one or more light beams from sources 310 (as previously described) directed through optically transmissive surfaces 306. Light from sources 310 is projected onto the sample at a fixed angle of about 45 degrees to the axis of sample holder 308, and is either scattered by the particles to sensor 312 or travels through the sample holder 308 and exits the through the back surface. Scattered light is detected by sensor 312, which preferably comprises a CCD camera and associated optics, for example as previously described. Black body chambers 314 are disposed opposite the light sources and sensor. Preferably these are separate chambers.

The immersion of the sample allows the illumination to penetrate sample holding device 308 without being refracted from the glass solution, even at angles above the critical angle of 42 degrees. Another benefit of the solution is that it reduces background scatter from dust particles. In a closed system, the solution can actually be pumped into tank 302 and re-circulated through a filter to remove any contamination from holding device 308. A prism tank permits illumination of the sample at a 45-degree angle to the lens, perpendicular to one of the tank walls. Note that the angle of illumination can be any angle, not just 45 degrees, as long as it does not enter the detection lens. The exiting light passes out the opposite wall, while sensor 312 images through two walls perpendicular to sample holding device 308. The exiting light from the illumination is shielded from the opposite side of the sensor wall to reduce unwanted background.

In preferred embodiments, tank 302 is arranged as a polygon, preferably to provide a hexagonal or octagonal chamber 304, with at least three optically transmissive surfaces. The hexagonal shape prevents the light from being scattered or deflected as it passes through chamber304, across sample holder 308, and back out through chamber 304 until it exits on the far side. The entrance and exit of the illumination beam is perpendicular to the tank surface, and is well out of the view of sensor 312, therefore, scattering at these two interfaces is not introduced to the sensed image.

As shown in FIG. 8, in further preferred embodiments, chamber 304 is a thin chamber, configured to have an internal width substantially less than the length of sample holding device 306. Two prisms are used to deliver light to the array, and transmit the image to the camera. Such a chamber allows illumination and detection in a liquid without using a large volume of liquid in the chamber and minimizes the potential of scattering from any particles in the liquid.. Reflective surface 316 permits redirection of excess illumination 318 and increases sensitivity of sensor 312 to scattered light 320

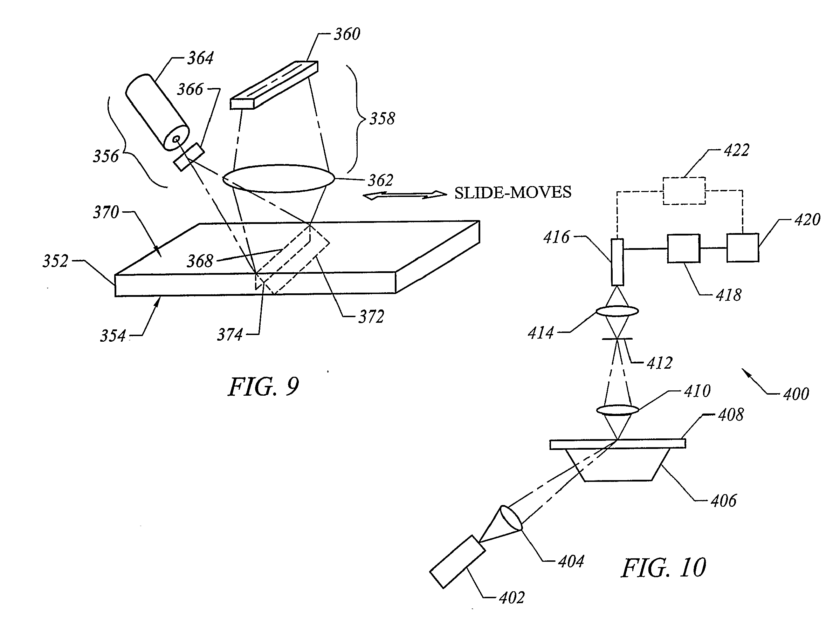

Referring to FIG. 9, a further alternative reader instrument 350 is designed to scan a single substrate, such as a 1x3 inch slide or other substrate 352 labeled with RLS particles. Substrate 352 may be supported by conventional means (not shown) that permit translation and precise positioning of the substrate. Instrument 350 confers an ability to reject artifacts located on backside 354 of substrate 352. Reader instrument 350 includes linear illumination assembly 356 and detection assembly 358. Detection assembly includes preferably CCD camera 360 and imaging lens 362, similar to that described above. However, camera 360 is, in this embodiment, a linear CCD camera, or alternatively a CMOS sensor for detecting a focused line of scattered light. The illumination assembly includes, for example, illumination source 364 for generating light and line generator 366, such as a cylindrical lens, for producing a line of light 368 from the illumination source. Illumination sources as described above may be utilized, but in this embodiment, a laser light source is preferred.

Linear illumination assembly 356 is oriented at an angle, e.g., approximately 45 degrees, with respect to the substrate surface 370. Detector 360 is focused on top surface 370 of slide 352 using imaging lens 362. Preferably, the illumination is centered along primary illumination line 368 so that the surface being measured has the maximum illumination intensity. Because the illumination entrance 372 (or exit) on the opposite surface is out of view of the line segment 374 captured by detector 360, light scattered by debris or artifacts on the backside 354 of slide 352 are not imaged. The entire slide is scanned preferably by moving slide 352 through the illumination and detection field. In alternative embodiments, substrate 352 is relatively stationary and the illumination assembly 356 and detection assembly 358 are moved linearly along the substrate.

In a further embodiment of assembly 356, the detector may have multiple rows along the length and employ the use of a prism or diffraction grating to split the incoming light from the sample into discrete spectra. In this approach, spatial information is captured in the long axis of the detector and spectral information in the short axis. For each measurement point, two or more spectra can be captured and further used to determine multiple labels within each location. For this approach, a multi-wavelength laser or broad-band slit source lamp would be employed.

The foregoing embodiments of the present invention offer many advantages in accuracy and flexibility of experiments that may be completed. This is particularly true with respect to applications involving RLS particle signals from intermediate and high-density arrays. However there are many interesting applications where there are only 10 to 20 spots in the microarray. For example, research laboratories developing clinical methods for detecting antigens that have so far been identified for specific cancers may use only a few spots representing validated clinical markers. For such low density arrays labeled with RLS particles, a simple light scattering detection system in which each of the spots are scanned manually across a relatively inexpensive photodiode or photomultiplier (PM) detector provides a low cost instrument that widens the number of laboratories that can use RLS detection systems. This makes RLS technology affordable to laboratories that wish to experiment with and develop applications for this signal generation and detection technology but cannot afford more expensive instrumentation. Moreover, the potentially lower cost and portable nature of such RLS devices make them appropriate devices for use outside the laboratory, i.e., field testing.

Furthermore, a dark field microscope could be used for RLS applications such as immunohistochemistry, in situ hybridization, and study of cell surface receptors. In such applications, relatively higher levels of magnification, for example 20-100 X or higher, may be used. Additionally, trans-illumination configurations would typically be employed with the illumination source disposed on the opposite side of the sample from the detector.

In general, the spots in present day microarrays have diameters ranging from 20 microns to 1 or serveral mm depending on whether the spots are deposited with a spotting instrument or by hand pipetting. However, spots with diameters less than 1mm can be formed and may be advantageous in some applications. Furthermore, the spatial distribution of RLS particles in a spot may be inhomogeneous (i.e., the particles may not be distributed evenly throughout the spot) and the spots in a specific microarray may not all have exactly the same size. A nonimaging instrument for measuring scattered light intensity from spots in a microarray preferably would address all of these error generating aspects of a microarray

In order to be most useful in such low cost and low intensity arrays, an instrument should exhibit a number of properties or characteristics. First, the instrument should be able to measure the scattered light intensity of each individual spot in a microarray without interference from other spots. The microarray spot diameter can range from 20 microns to 1 or several mm and with edge to edge separations or spacing between the spots of 200 micron or less. Second, the instrument should be able to measure the integrated light intensity from a whole spot (intensity from the whole spot and not only a small area of the spot) to minimize (a) the effects of inhomogeneities in the spatial distribution of RLS particles and (b) distribution of spot diameters in a given array. Next, the instrument should have the ability to detect integrated light intensity over a large intensity range, preferably four decades of light intensity. In addition it should have high sensitivity so as to be able to measure the scattered light intensity of spots with particle densities down to about 0.005 particles/$m^2$. It should also be easy to align each individual spot in the light sensing area of the detector.

Thus, in a further alternative embodiment of the invention, a relatively low cost instrument 400 uses a dark field microscope or optics in combination with a photodiode or photomultiplier (PM) tube for detection. As shown in FIG. 10, instrument 400 includes an illumination source 402 providing a light beam focused through objective lens 404 and prism 406 onto substrate holder 408 to create a dark field image. In certain versions the prism can be eliminated. Any illumination source may be employed as previously described, however, an optical fiber source may provide advantages in smaller devices. Second objective lens 410 is disposed opposite the prism with respect to sample holder 408 to focus the image on image plane 412. Eyepiece lens 414 focuses the image into detector 416. Detector 416 is preferably a photodiode, avalanche photodiode, or photomultiplier tube. Detector 416 preferably communicates with a sensitive, but relatively inexpensive current to voltage converter 418 to measure the electrical current signal from the photodiode or PM tube. Digital voltmeter 420 reads the intensity voltage signal from current to voltage converter 418. Digital voltmeter 420 may be a commercially available and relatively inexpensive component.

A preferred current to voltage converter comprises a conventional low noise LF 441 operational amplifier with the following characteristics: Input Bias Current -- 50 pA, Input Noise Current -- 0.01 pA/$\sqrt{Hz}$, Power Supply Current-1.8 mA, Input Impedance -- $10^{12}$ ohms, and Internal Trimmed offset Voltage -- 0.5 mV. A preferred photodiode is the PIN-5DP photodiode (UDT Sensor Inc.) which is an ultra low noise, low frequency photodiode optimized for photovoltaic operation. Its important characteristics are as follows: Detection Area -- 5.1 $mm^2$ (2.6 mm diameter circular area), Responsivity -- 0.12 A/ W(400 nm), 0.4 A/W (632 nm), 0.6 A/W (970 nm), and Noise Equivalent Input -- 3.4 x $10^{-15}$ W/ $\sqrt{Hz}$. A preferred photomultiplier tube is the 1P28 PM tube, which has the following characteristics: Spectral Response Range -- 185 to 680 nm, Number of Dynodes-9, Tube Diameter -- 1-1/8, Max Anode Current --100 $\mu$A, Max Anode to Cathode Voltage -- 1250 V, Anode Sensitivity -- 2.4 x $10^5$ A/W, and Current Amplification (1000 VDC) of 5 x $10^6$ and an Anode Dark Current of 5 nA for 1000V Anode to Cathode Voltage.

To obtain a measurement, a low-density microarray glass or plastic slide (or even small well plate) is disposed on sample holder 408. Each spot or well in the array is then scanned manually through the field of view of the microscope objective. Detector 416 detects an integrated RLS intensity from each spot. The current signal from detector is converted to a voltage signal that is read by digital voltmeter 422. The end result is the integrated intensity of each spot expressed as voltage.

In its most basic form, instrument 400 does not require a computer or expensive software. An alternative form of instrument 400 employs optional alternative computer 422 to read the signals directly from detector 416 or through one of the other electronics components. Since the transfer of massive image data is not required, the computerized version of instrument 400 may use an inexpensive computer with an analog to digital converter board to digitize the signal from the photodiode or PM tube. The required software may be provided by a person of ordinary skill in the art based on the teachings herein. Another possibility is to use a computer chip with embedded software in place of the computer.

As described above, instrument 400 uses a dark field microscope and a photodiode attached to an eyepiece of the microscope. In another form of the instrument, a microscope is not used. Instead, microscope objective 410 and eyepiece 414 are mounted in a tube and detector 416, such as a photodiode or PM tube, is attached to eyepiece lens 414. The latter allows construction of an even simpler and still less expensive instrument

To utilize instrument 400, a spot is manually positioned in front of second objective lens 410, which forms a magnified image of the spot at image plane 412 (typically about 160 mm from the shoulder of the objective). The power (magnification) of objective lens 410 (e.g., 4x, 10x, etc.) is preferably chosen such that (1) when the spot is viewed through the microscope eyepiece, the spot occupies as much of the field of view of the objective (as viewed through the eyepiece) as possible without exceeding the field and (2) other spots in the microarray are not in the field of view. This arrangement essentially isolates one spot from the rest of the spots in the microarray and minimizes the background signal from areas outside of the spot. For example, a 300 micron diameter spot viewed with a 40 x objective has a diameter of 12 mm at the image plane of the objective. The diameter of the light sensitive area of an exemplary photodiode (PIN-5DP) is about 2.6 mm. (Photodiodes with larger sensing areas are available.) If this photodiode is placed at the center of the magnified image, it would detect intensity only from a small area of the 12 mm diameter image which would correspond to a small area on the 300 micron spot in the array. With this arrangement, the photodiode would not detect the integrated light intensity from the spot but would only measure the intensity from a small area of the spot. The measured light intensity would thus be subject to errors of variations in spot diameter and inhomogeneities.

To get around these problems, the magnified image is demagnified using a 10x objective that focuses the magnified image down to a spot that has a diameter that is less than 2 mm.. The sensitive area of the photodiode is then positioned on the demagnified, focused spot. Since the focused spot is smaller than the photodiode sensitive area (about 2.6 mm diameter), the photodiode thus measures the integrated scattered light intensity from the whole spot. In instrument 400, objective 410 thus serves to isolate a specific spot from other spots in microarray and eyepiece 414 serves to demagnify the magnified image so that the photodiode detector measures the intensity from the whole spot.

As described above, a 300 $\mu$m diameter spot was used as an example. For spots of other sizes, the measurement logic is the same except that one selects an objective whose field of view is just larger than the dimensions of the spot but not much larger. The following table shows the field of view of objectives with different powers (magnifications).

## TABLE 1

| Field of View of Objectives with Different Powers | |
| --- | --- |
| Objective Power | Field of View Diameter (mm) |
| 4x | 4.5 |
| 10x | 1.8 |
| 20x | 0.9 |
| 40x | 0.45 |
| 60 | 0.30 |
| 100x | 0.18 |

The size of the demagnified image on the face of detector 416 is not very sensitive to the power of objective 410 and the demagnified image remains within the sensitive area of the detector for all objective powers. (The 10x ocular used for demagnification is a wide field ocular which comes with the Fisher Micromaster I Microscope--with trinocular body--that is used in the Dark Field Microscope. Other oculars can, of course, be used.)

Instrument 400 as describe above preferably uses a conventional dark field microscope. A dark field microscope typically consists of a viewing port with two 10x eyepieces (one for each eye) and a detection port where the demagnifying eyepiece and photodiode are mounted. The eyepiece on the detection port is positioned at the same distance from the objective as the eyepieces in the viewing port. With this arrangement, the image and field of view seen in the viewing port is the same as in the detection port. Thus when the spot is correctly positioned and focused as seen through the viewing port, it is also correctly positioned for detection by the photodiode.

A procedure for measuring the integrated scattered light intensity from a specific spot using instrument 400 may be summarized as follows:

a. Place the microarray glass or plastic slide on the stage of the dark field microscope.
b. View the microarray with a x4 objective and manually move the slide until the desired spot is in the field of view of the eyepieces in the viewing port. Focus the spot and center it in the field of view.
c. Select an objective in which the spot is just smaller than the field of view.
d. Focus and center the spot.
e. Pull the lever on the microscope that shifts the viewed area to the detection port.
f. Read the signal from the photodiode with the high input impedance voltage meter.

To facilitate the manual movement of sample holder 408, a frame (not shown) may be provided to position the holder, which can easily be manipulated manually. The frame not only facilitates the manual movement of sample holder 408, but prevents the movement (through sliding) after a spot has been positioned in the field of view of objective lens 410.

A photomultiplier (PM) tube typically has a much larger detection surface than a photodiode (about 8 mm by 24 mm detection area for the 1P28 PM tube and larger for other PM tubes). The detection surface is thus sufficiently large for a PM tube to detect a significant portion of the magnified image produced by microscope objective 410 without having to demagnify the image. Thus, the detection surface of a PM tube can be positioned on image plane 412 and measure the scattered light intensity from a significant portion of the spot without demagnification. However, such an arrangement has a very large depth of field and may detect a large amount of stray light, thus resulting in a large background signal. To minimize stray light detection, it is beneficial to place an aperture (not shown) in front of the PM tube to confine the depth of field. The aperture placed at the image plane of the objective should have a diameter that is just larger than the magnified image. Alternatively, one can use the same arrangement as for the photodiode detector and detect the intensity of the demagnifed spot through a 3 mm aperture. This alternative method may be preferred because the optical arrangement would then be the same for photodiode and photomultiplier detection. It is therefore easy to change from photodiode detection to PM detection or vice versa merely by exchanging the detectors.

In practice, the same arrangement as the photodiode (demagnification with an eyepiece) may be used for detection with a PM tube. However, the PM tube itself is not placed on the demagnified image. Instead, the light from the image is picked up through a fiber optic light guide, which delivers the integrated image intensity to the PM tube. The entrance of the light guide (48 inches long, 3 mm diameter) is placed on the focused demagnified image produced by the eyepiece. The light at the exit of the optical guide is detected by the PM tube. The optical guide essentially acts as a 3 mm aperture at the focal point of the demagnified image of the spot and guides the light to the PM tube.

### Example I -- Measurement of Scattered Light Intensity with Photodiode Detector

The photodiode version of the detection system described above was used to measure 80 nm gold spots on a microarray. The microarray was prepared using the Cartesian spotter as follows: An 80 nm gold particle suspension with OD(554)=210 was diluted serial by factors of 2 using 1% gelatin, 25 % DMSO. These solutions were then used to spot 80 nm gold particles in a series of spots in which the gold particle density decreased by 2x from spot to spot. Spots were deposited with the Cartesian spotter and had a diameter of about 300 microns. The distribution of particles in the spots was very homogeneous as viewed in the dark field microscope. The spots in air and water displayed an yellow gold scattered light color instead of the usual greenish color. (In accordance with principles for modulating light scattering properties of RLS particles in environments of differing refractive index, in some arrays made from gelatin, the array displays green scattered light in water after washing, but is still orange in air. For arrays made using Ficoll, it was observed that the color in air and water is orange before washing, but after washing, the array scattered light is green in water and yellowish green in air.) In the present experiment, the fact that light scattering spectrum is perturbed by the gelatin is not of interest. The array is merely used as a source of spots with gold particles where the particle density is decreased serially by x 2. Some particles came off the spots when washed but the amount is not significant. The integrated scattered light intensity from each spot bathed in air was measured with the photodiode in the dark field microscope as described above. The spots were illuminated with white light. The following results were obtained:

**TABLE 2**

| Scattered Light Intensity vs. Particle Density Measured with Photodiode Connected to Op Amp Current to Voltage Converter with 50 M Feedback Resistor | | |
| --- | --- | --- |
| Particles/$\mu^2$ | Intensity, Volts | Intensity - Background |
| 8.19 | 42 | 41.86 |
| 4.1 | 26.6 | 26.46 |
| 2.05 | 14.57 | 14.43 |
| 1.02 | 8.89 | 8.753 |
| 0.512 | 6.41 | 6.273 |
| 0.256 | 3.51 | 3.373 |
| 0.128 | 2.12 | 1.983 |
| 0.064 | 1.14 | 1 |
| 0.032 | 0.76 | 0.623 |
| 0.016 | 0.69 | 0.553 |
| 0.008 | 0.39 | 0.253 |
| 0.004 | 0.308 | 0.171 |
| $^2$Dark | 0.0002 to 0.0012V | |
| $^3$Background | 0.137 | |

These data demonstrate that the detector described above is able to provide a high level of sensitivity as well as very good accuracy and linearity over a broad range of particle surface densities without exhaustive optimization and within the limits of experimental error. The lowest limit of sensitivity is determined by background stray light and not by photodiode detector dark current.

### Example II.A Measurement of Scattered Light Intensity from RLS Gold Particle Array Using a Load Resistor to Convert PM Current To Voltage (No Op Amp Current to Voltage Converter) -- Test of Linearity of 1P28 PM Tube Connected to a Load Resistor

In these measurements, the scattered light intensity was measured with the 1P28 PM tube as described above but instead of connecting the anode of the PM tube to the current to voltage converter it was simply connected through a load resistor RL to ground. The load resistor acts as a current to voltage converter. The voltage is read with a high input impedance digital voltmeter connected across the load resistor. The load resistor was attached externally (outside the PM housing) to the anode (signal out ) BNC connector.

The use of a simple load resistor in place of the current to voltage converter is a very simple and inexpensive method for measuring the current signal from the PM tube and is made possible by the high amplification (around 5 x $10^5$ for 650 high voltage) capabilities of the PM tube. The voltage across the load resistor is related to the current Ia at the PM tube anode by the expression

$$V = Ia \times RL$$

Although the use of a load resistor for current to voltage conversion is very simple, it is subject to the following limitation (as well as the limitations of anode current described in a previous section). In normal operation, there is a voltage difference Vad between dynode 9 (last dynode) and the PM anode. This voltage is responsible for the collection at the PM anode of the signal electrons produced at the ninth dynode. This voltage difference is provided by the dynode resistor network and is given the expression

$$Vad = In \times 3.3 \times 10^5$$

where In is the current through the chain resistor network and assuming that the anode at ground (zero) voltage. $3.3 \times 10^5$ is the value of the chain resistor between dynode 9 and ground. The amplification of the PM tube is sensitive to changes in the value of Vad. When the anode current is measured through an op amp current to voltage converter, the anode is kept at ground voltage (zero voltage) even when current is flowing through the anode resistor. However, when the current is measured by a load resistor connected to the anode, the anode is no longer at ground voltage but has a voltage equal to the voltage across the load resistor. High PM currents such as 10 μA produce a voltage drop of 10V across a 1 M anode load resistor. This voltage then diminishes the voltage between the ninth dynode and anode by 10 V, which may result in a nonlinear response at PM high currents.

The light intensity detected by the PM tube is kept below PM currents that do not produce nonlinear responses. One method for determining the PM currents at which the PM response becomes nonlinear is to illuminate the PM tube so as to produce say 10 V across the anode load resistor RL. Then introduce a 1 OD neutral density filter (x10 light attenuator) into the illumination path and observe whether the voltage across RL drops by x10. Using this method, the maximum voltage across RL which is in the linear range can be determined. The following table shows results obtained using the latter method to determine max RL voltage which is in linear region. In these measurements RL was 1 M. Dark current was 0.8 mV.

**TABLE 3**

| Determination of Max RL Voltage which is in the Linear Range When Signal is Measured Directly Across RL using A High Impedance Voltmeter | | |
|---|---|---|
| RL Voltage (0 OD), volts | RL Voltage (1 OD), volts | *Ratio |
| 8.5 | 0.640 | 13 |
| 3.96 | 0.311 | 13 |
| 1.4 | 0.124 | 11.2 |
| 0.74 | 0.0645 | 11.5 |
| *Ratio is RL Voltage (0 OD)/RL Voltage (1 OD). Signal across RL was measured with a Wavetek DM78 high impedance digital voltmeter. | | |

The results of the above table show that the PM signal voltage across RL is linear up to an RL voltage of at least 8.5 V assuming that the about 13 % differences of the ratios in the table are due to experimental error in positioning the neutral density filters. The anode current restrictions for a 1P28 PM tube are summarized as follows:

a. The anode current should not exceed 1/20 of the current through the dynode resistor network.

b. The anode current should not exceed 100 μA at 1000 V of PM negative high voltage.

c. When measuring PM current with a load resistor rather than with a current to voltage converter, the voltage across the load resistor should not be greater than about 8 to 10 volts.

It should be noted that as described above for the photomultiplier tube, the photodiode photocurrent can also be measured with a simple load resistor instead of an op amp current to voltage converter. However, here again, the voltage produced across the resistor by the photocurrent produces a voltage that opposes the photocurrent from the photodiode and may introduce a nonlinear response.

**Example II.B Measurement of Scattered Light Intensity from RLS Gold Particle Array using a 1P28 PM Tube and High Impedance Digital Voltmeter Connected to Anode Load Resistor**

The following table shows values of scattered light intensity vs particle density obtained using a 1P28 PM tube connected to a 1 M anode load resistor. The voltage signal across the load resistor was measured with an inexpensive Wavetek DM78 high input impedance digital voltmeter. The array used in these measurements is the same used above for the PIN 5 DP photodiode connected to an op amp current to voltage converter.

**TABLE 4**

| Scattered Light Intensity vs Particle Density Measured with a 1P28 PM Tube, Anode Load Resistor RL of 1 MΩ and High Input Impedance Voltmeter Connected Directly to Anode Load Resistor (OD=1 Neutral Density Filter in front of PM Tube) | | |
| --- | --- | --- |
| Particles/m$^2$ | Intensity,RL=1 MΩ, (Volts) | Intensity-Bckgrd |
| 8.19 | 8.78 | 8.74 |
| 4.1 | 6.17 | 6.13 |
| 2.05 | 3.35 | 3.31 |
| 1.02 | 2.07 | 2.03 |
| 0.512 | 1.53 | 1.49 |
| 0.256 | 0.874 | 0.834 |
| 0.128 | 0.458 | 0.418 |
| 0.064 | 0.261 | 0.221 |
| 0.032 | 0.188 | 0.148 |
| 0.016 | 0.142 | 0.102 |
| 0.008 | 0.095 | 0.055 |
| 0.004 | 0.0727 | 0.0327 |
| [2]Dark | 0.0008 | |
| [3]Background | 0.04 | |

The above data show that the described detector provides a high level of sensitivity as well as very good accuracy and linearity over a broad range of particle surface densities without exhaustive optimization and within the limits of experimental error. The lowest limit of sensitivity is determined by stray background light and not by PM detector dark current. The PM tube has a much higher light detection sensitivity than a photodiode. A neutral optical density filter with OD=1 was therefore placed in front of the PM tube to keep the voltage signal across the PM anode resistor to less than about 9 volts for the highest array signal.

**Example III -- Measurement of Scattered Light Intensity from RLS Gold Particle Array using a 1P28 PM Tube with Anode Connected to an Op Amp Current to Voltage Converter**

The following table shows values of scattered light intensity vs particle density obtained using a 1P28 PM tube connected to an op amp current to voltage converter. The array used in these measurements is the same as used above for the PIN 5 DP photodiode connected to an op amp current to voltage converter and 1 P28 PM tube connected to a load resistor.

**TABLE 5**

| Scattered Light Intensity vs Particle Density Measured with PM Tube and Op Amp Current to Voltage Converter (OD=2 Neutral Density Filter). RL Refers to the Value of the Feedback Resistor in the OP Amp Feedback | | | | |
|---|---|---|---|---|
| Particles/m$^2$ | Intensity, RL=50 MΩ, Volts | Intensity, RL=5 MΩ, Volts | [3]Intensity, Adjusted to 50 MΩ | Intensity - Bkdground |
| 8.19 | | 6.36 | 61.49 | 61.19 |
| 4.1 | | 4.75 | 45.92 | 45.62 |
| 2.05 | | 2.9 | 28.04 | 27.74 |
| 1.02 | | 1.62 | 15.66 | 15.36 |
| 0.512 | | 1.162 | 11.23 | 10.94 |
| 0.256 | 6.69 | 0.692 | 6.69 | 6.39 |
| 0.128 | 3.52 | | 3.52 | 3.22 |
| 0.064 | 1.84 | | 1.84 | 1.54 |
| 0.032 | 1.388 | | 1.388 | 1.09 |
| 0.016 | 1.02 | | 1.02 | 0.723 |
| 0.008 | 0.704 | | 0.704 | 0.407 |
| 0.004 | 0.544 | | 0.544 | 0.247 |
| [1]Dark | 0.041 | | | |
| [2]Background | 0.297 | | | |
| [1]Shutter to PM Tube closed. [2]Shutter open and microscope focused on an area of the microarray slide that does not contain a spot. [3]The conversion factor for converting from the 5 M to the 50 M scale was obtained from the intensities at 0.256 particles/micron$^2$ which yields the factor 6.69/0.692=9.667. | | | | |

The above data show that the described detector provides a high level of sensitivity as well as very good accuracy and linearity over a broad range of particle surface densities without exhaustive optimization and within the limits of experimental error. The lowest limit of sensitivity is determined by stray background light and not by PM detector dark current. The PM tube-Current to Voltage Converter combination has a very high light detection sensitivity. A neutral optical density filter with OD=2 was therefore placed in front of the PM tube to keep the Current to Voltage Converter from saturating.

**Example IV -- Comparison of Results Obtained by the Three Detection Methods Described Above**

Two ways were used to compare the intensity vs. particle density data obtained by the three methods described above to determine how well the data obtained by the three methods are correlated. The following symbols are used. INTENSITY PMOPAMP= Scattered light intensity measured with 1P28 PM tube connected to op amp current to voltage converter.
INTENSITY PMRL = Scattered light intensity measured with 1P28 PM tube connected to load resistor.
INTENSITY PHOTODIODE = Scattered light intensity measured with PIN 5DP Photodiode connected to op amp current to voltage converter.

The methods are as follows:

1. Method 1

In this method Intensity measured by one detector vs. Intensity measured by one of the other detection modes is plotted. If the correlation between the two modes of detection is good, then the points in the plot should be on a straight line. Visual inspection of natural and log-log plots of INTENSITY PMOPAMP VS INTENSITY PMRL showed that the points in the graph fall on a straight line. Visual inspection of natural and log-log plots of INTENSITY PHOTODIODE vs INTENSITY PMRL showed that the points in the graph fall on a straight line.

2. Method 2

In this method the intensities INTENSITY PMRL are normalized to the intensities INTENSITY PMOPAMP and the intensities are compared. The normalization is performed as follows. First the graph INTENSITY PMOPAMP VS INTENSITY PMRL is curve fitted, yielding the expression INTENSITY PMOPAM = 7.186* INTENSITY PMRL

Next the intensities INTENSITY PMRL are multiplied by 7.186. This essentially normalizes the PMRL intensity values to the PMOPAMP intensity values. Finally the percent difference from the intensities INTENSITY PMOPAMP and 7.186*Intensity PMRL is calculated using the following expression PERCENTDIFF = $\underline{\text{(INTENSITYPMOPAMP - 7.186 X INTENSITYPMRL) *100}}$ INTENSITYPMOPAMP

Visual inspection of plots of INTENSITY PMOPAMP and 7.186*INTENSITY PMRL vs. Particle Density showed that the agreement between the two sets of data is very good. It should be noted from these plots that the fluctuations in intensity about an imaginary line through the points is the same for both sets of data indicating that the fluctuations are in the microarray and not in the detection systems.

Those in the art will recognize that the methods and apparatus described herein have broad utility. They can be applied in one form or another to most situations where it is desirable to use a signal generation and detection system as part of an assay system to quantitate and/or detect the presence or absence of an analyte. Such analytes include industrial and pharmaceutical compounds of all types, proteins, peptides, hormones, nucleic acids, lipids, and carbohydrates, as well as biological cells and organisms of all kinds. One or another mode of practice of this invention can be adapted to most assay formats which are commonly used in diagnostic assays of all kinds. For example, these include heterogeneous and homogeneous assay formats that are of the sandwich type, aggregation type, indirect or direct and the like. Sample types can be liquid-phase, solid-phase, or mixed phase.

Those in the art will recognize that the apparatus described herein have broad utility. They can be applied in one form or another to most situations where it is desirable to use a signal generation and detection system as part of an assay system to quantitate and/or detect the presence or absence of an analyte. Such analytes include industrial and pharmaceutical compounds of all types, proteins, peptides, hormones, nucleic acids, lipids, and carbohydrates, as well as biological cells and organisms of all kinds. One or another mode of practice of this invention can be adapted to most assay formats which are commonly used in diagnostic assays of all kinds. For example, these include heterogeneous and homogeneous assay formats, which are of the sandwich type, aggregation type, indirect or direct, and the like. Sample types can be liquid-phase, solid-phase, or mixed phase.

The following section of the description consists of numbered paragraphs simply providing statements of the invention already described herein. The numbered paragraphs in this section are not claims. The claims are set forth below in the later section headed "claims".

1. An apparatus for light scattering particle label analysis, comprising:

a substrate holder adapted to hold a substrate presenting a sample for analysis;

an illumination system comprising a light source directed at said substrate holder and a sample presented therein; and

a scattered light detection system comprising a light detector cooperating with said substrate holder and illumination system to detect light scattered from particles in the sample.

2. The apparatus of 1, wherein:

said substrate holder is configured to hold at least two different sample presentation substrates, the different substrates having different illumination area requirements; and

said illumination system comprises a variable aperture configured to generate the illumination area required for each different substrate.

3. The apparatus of 1 or 2, wherein said substrate holder comprises a plurality of removable inserts, each configured to hold different substrates.

4. The apparatus of 3, wherein said substrate is selected from the group consisting of chips, slides, microtiter plates, membrane carriers, test tubes, capillary tubes, flow cells, microchannel devices, cuvettes, dipsticks, containers for holding liquid or solid phase samples.

5. The apparatus of any one of 1-4, wherein said illumination system further comprises an aperture and focusing optics such that light passing through said aperture is focused with a profile and area shaped to match an illumination area of the substrate.

6. The apparatus of 5, wherein said aperture comprises an element defining at least one opening of a first diameter for entry of light, said element being rotatably mounted to vary the opening to the light.

7. The apparatus of 6, wherein said element defines plural openings, each providing an aperture corresponding to a selected substrate illumination area.

8. The apparatus of 7, wherein said aperture further comprises: a motor; a shaft extending from the motor with the element mounted thereon; and an encoder cooperating with said element to determine the angular position of the element and openings.

9. The apparatus of 6, wherein said element comprises a drum, said drum defining at least opening of a second larger diameter opposed to said first diameter opening, the second opening permitting exit of light.

10. The apparatus of 9, wherein said drum defines plural entry and exit openings, each paired to provide an aperture corresponding to a selected substrate illumination area.

11. The apparatus of 5, wherein said profile and area match a flat bottom illumination area of a microtiter plate well, or wherein said profile and area match a polygonal illumination area of an array, or wherein said profile and area match a circular area of microplate wells.

12. The apparatus of any one of 1-11, wherein said substrate holder and image detection system cooperate to scan at least a portion of a substrate to provide a plurality of sub-images, said plurality of said sub-images being combined to form a composite image.

13. The apparatus of 12, further comprising a processor communicating with the image detection system to generate said composite image.

14. The apparatus of any one of 1-13, further comprising a control system communicating with the substrate holder, illumination system and detection system.

15. The apparatus of any one of 1-14, wherein: said substrate holder comprises X and Y stages for precisely positioning the substrate with respect to the illumination system for creation of plural image tiles to be assembled into a composite image; said substrate holder includes an imaging target disposed in a plane the image to be detected; and said detection system captures images at two or more locations including said imaging target, allowing calibration of the movement of the X and Y stages for precisely assembling each image tile into the composite image.

16. The apparatus of any one of 1-15, wherein said illumination system comprises at least one light source directed at an optically transmissive fluid filled tank, with said substrate holder being disposed therein.

17. The apparatus of 16, wherein the fluid in said tank and an optically transmissive portion of said tank have refractive indexes that at least approximately match.

18. The apparatus of any one of 1-17, wherein said illumination system comprises a plurality of light emitting diodes (LEDs) focused on a target illumination area.

19. The apparatus of 18, wherein said LEDs are supported in a hollow cylindrical housing adapted to be placed over an objective lens of the light detection system.

20. The apparatus of 19, wherein said housing defines a narrowed portion configured and dimensioned to reduce entry of extraneous light to the detection system.

21. The apparatus of any one of 1-20, wherein said illumination system comprises a light source producing a line of light along an illumination area on the substrate and said detection system includes a sensor for detecting a focused line of light.

22. The apparatus of 21, wherein the light detector has a field of view and said light source is configured and dimensioned such that the illumination line is presented to the sample at an angle selected to cause light exiting the substrate opposite the sample to be outside the field of view of the light detector.

23. The apparatus of any one of 1-22, wherein said detection system comprises a photomultiplier, photodiode or a charge coupled device, or wherein said detection system further comprises multiple magnification detection lenses.

24. The apparatus of any one of 1-23, wherein said light source is a tunable light source.

25. The apparatus of any one of 1-24, wherein said illumination system comprises a broad- band light source and said apparatus further comprises a plurality of individually selectable spectrally discriminative light filters disposed in at least one of the illumination system or detection system, or wherein said illumination system comprises a broad-band light source and said apparatus further comprises at least one tunable LCD spectrally discriminative light filter disposed in at least one of the illumination system or detection system.

26. The apparatus of any one of 1-25, wherein two or more magnifications are utilized to capture integrated intensity values from areas of interest at low magnification, then perform particle counting at higher magnifications.

27. The apparatus of 26, wherein integrated intensity and particle counting routines are performed using an automated software routine which combines the data from integrated intensity and particle counting to increase measurable range of the label.

28. A multiformat analyte assay system, comprising:

a substrate holder configured and dimensioned to accept any of a plurality of different format sample presentation devices; and
an analyte detection system cooperating with said substrate holder,
wherein said detection system is configurable to detect analytes in a sample presented on any of said plurality of different format sample presentation devices.

29. The multiformat analyte assay system of 28, wherein said plurality of different format sample presentation devices comprise at least two of chips, slides, microtiter plates, membrane carriers, test tubes, capillary tubes, flow cells, microchannel devices, cuvettes, dipsticks, containers for holding liquid or solid phase samples.

30. The multiformat analyte assay system of 29, wherein said analyte detection system is one of a light scattering system, a fluorescent system, a luminescent system, a chemiluminescent system or anelectrochemiluminscent system.

31. The multiformat analyte assay system of 30, wherein said holder accepts any of a plurality of different inserts that are configured to hold different sample presentation devices.

32. The multiformat analyte assay system of 29, wherein said detection system comprises:

an illumination system comprising a light source directed at said substrate holder and a sample presented therein; and

a scattered light detection system comprising a light detector cooperating with said substrate holder and illumination system to detect light scattered from particles in the sample.

33. The multiformat analyte assay system of 32, wherein said illumination system further comprises a variable aperture configured to generate an illumination area corresponding to illumination requirements for each different sample presentation device.

34. An apparatus for light scattering particle label sample analysis, comprising:

an illumination system comprising an annular ring light source; and
a scattered light detection system comprising a light detector, wherein,
when a sample containing substrate is present in said apparatus, said light source provides light to the sample, and said detector detects light scattered from any light scattering particles associated with said sample.

35. The apparatus of 34, wherein said annular ring light source comprises a light emitting diode (LED) ring, and/or wherein said light source is a tunable light source comprising LEDs producing different color light.

36. An apparatus for light scattering particle label sample analysis, comprising:

an illumination system comprising a light source;

an immersion tank sample device chamber; and

a scattered light detection system comprising a light detector, wherein, when a sample device is present in said sample device chamber, said light source provides light to said sample device, and said detector detects light scattered from any light scattering particles associated with a sample on said sample device.

37. An immersion tank sample device chamber configured for a light scattering particle label sample analyzer, comprising:

a plurality of adjoining surfaces providing a liquid-containing structure,
where at least one said surface is an optically transmissive surface;
a refractive index matching liquid in said structure; and
a sample device with light scattering particle labels bound thereto,
wherein said sample device is immersed in said liquid and said light scattering labels can be illuminated by a light beam directed through said optically transmissive surface.

38. The sample device chamber of 37, wherein the chamber comprises a polygon with at least 3 optically transmissive surfaces.

39. The sample device chamber of 38, wherein said polygon is an octagon.

40. The sample device chamber of 37, wherein said chamber is configured to have an internal width substantially less than the length of said sample device.

41. An apparatus for light scattering particle label analysis, comprising:

an illumination assembly disposed on a first side of a substrate support and configured to produce a line of light on a substrate carried by the support; and

a detection system disposed above the substrate on the first side, said detection system being configured to detect a focused line of scattered light.

42. The apparatus of 41, wherein the illumination assembly comprises a light source and cylindrical lens configured to focus a line of light along a top surface of the substrate.

43. The apparatus of 42, wherein the detection system comprises detector focused on the top surface of the substrate and defines a field of view extending into the substrate and terminating before the opposite surface thereof.

44. An apparatus for light scattering particle label analysis, comprising:

a substrate holder;

an illumination system configured to focus a light beam on a first side of a substrate disposed in the substrate holder thereby creating a dark field image;

a detection system configured to view the dark field image on an opposite, second side of the substrate holder in the substrate holder, said detection system including a photo-detector producing a voltage based signal in response to scattered light intensity.

45. The apparatus of 44, wherein said photo-detector comprises a photodiode or photomultiplier tube communicating with a voltage converter and a voltmeter to read voltage signals from current signals input to the voltage converter by said photodiode or photomultiplier tube.

46. The apparatus of 44, wherein: the illumination system includes an objective lens disposed between a light source and the substrate holder; and the detection system includes an objective lens disposed between the photo- detector and the substrate holder to provide an image plane in front of said photo- detector.

47. An apparatus for light scattering particle label sample analysis, comprising:

means for holding a substrate containing a sample to be analyzed;

means for illuminating the sample; and

means for detecting light scattered by particles present in said sample.

48. The apparatus of 47, wherein said means for holding comprises an open structure adapted to receive a plurality of different sample presentation devices and position said devices on an imaging plane, each said different sample presentation device having a different illumination area.

49. The apparatus of 48, wherein said open structure is mounted on a carriage and said carriage is mounted on first and second stages for translation in two dimensions along said imaging plane.

50. The apparatus of 48, wherein said means for illuminating comprises a light source focused on said imaging plane and configured to provide different illumination areas corresponding to the sample presentation devices.

51. The apparatus of 50, wherein said means for illuminating further comprises a variable aperture configured to generate the corresponding illumination area for each different sample presentation device.

52. The apparatus of 51, wherein said variable aperture comprises a rotatable element defining plural entry and exit openings, said entry openings being of a first diameter and for entry of light and said exit openings being of a second larger diameter for exit of light, said entry and exit openings being paired to provide an aperture corresponding to a selected sample presentation device illumination area.

53. The apparatus of 52, wherein said element is a drum.

54. The apparatus of any one of 47-53, wherein said means for detecting comprises a CCD camera receiving scattered light from the sample.

55. The apparatus of any one of 47-54, wherein:

the means for illuminating comprises an annular ring light source; and

the means for detecting comprises a light detector.

56. The apparatus of 55, wherein said annular ring light source comprises a light emitting diode (LED) ring.

57. The apparatus of 56, wherein said light source is a tunable light source comprising LEDs producing differently colored light.

58. The apparatus of any one of 47-57, wherein:

the means for illuminating comprises a light source and an immersion tank surrounding said means for holding a substrate; and

the means for detecting comprises a light detector.

59. The apparatus of 58, wherein:

the immersion tank comprises a plurality of adjoining surfaces providing a liquid-containing structure, with at least one said surface is an optically transmissive surface;

a refractive index matching liquid is contained in said tank; and

the substrate is immersed in said liquid by the means for holding and light scattering particles in a sample on said substrate are illuminated by a light beam directed from the light source through said optically transmissive surface.

60. The apparatus of 59, wherein said tank comprises a polygon with at least 3 optically transmissive surfaces, or wherein said tank is configured to have an internal width substantially less than the length of said substrate.

61. The apparatus of any one of 47-60, wherein:

the means for illuminating is disposed on a first side of the means for holding and is configured to produce a line of light on a substrate carried by the means for holding; and
the means for detecting is disposed above the substrate on the first side,
said means being configured to detect a focused line of scattered light.

62. The apparatus of 61, wherein means for illuminating comprises a light source and cylindrical lens configured to focus a line of light along a top surface of the substrate.

63. The apparatus of 62, wherein the means for detecting comprises a light detector focused on the top surface of the substrate defining a field of view extending into the substrate and terminating before the opposite surface thereof.

64. The apparatus of any one of 47-63, wherein:

the means for illuminating is configured to focus a light beam on a first side of a substrate disposed in the means for holding thereby creating a dark field image;

the means for detecting is configured to view the dark field image on an opposite, second side of the substrate holder in the substrate holder; and

the means for detecting includes a photo-detector producing a voltage based signal in response to scattered light intensity.

65. The apparatus of 64, wherein said photo-detector comprises a photodiode or photomultiplier tube communicating with a voltage converter and a voltmeter to read voltage signals from current signals input to the voltage converter by said photodiode or photomultiplier tube.

66. The apparatus of 64, wherein:

the means for illuminating comprises an objective lens disposed between a light source and the means for holding; and

the means for detecting comprises an objective lens disposed between the photo-detector and the substrate holder to provide an image plane in front of said photo-detector.

**Claims**

1. A method for normalizing assay data, comprising:

selecting a first population of assay data and a second population of assay data, wherein said first population comprises a dependent set of controls and
said second population comprises an independent set of controls;
obtaining a linear relationship between said independent set of controls and
said dependent set of controls; and
applying said linear relationship to said first population, thereby producing normalized assay data.

2. The method of claim 1, wherein said first set of controls and said second set of controls comprise spots in an array, or wherein said first set of controls and said second set of controls are treated as equivalent.

3. The method of claim 1 or 2, wherein said linear relationship has a slope, m, and an intercept, b.

4. The method of any one of claim 1- 3, wherein said obtaining comprises applying linear regression to said controls, wherein assay data for said first set of controls is treated as a dependent variable and assay data for said second set of controls is treated as an independent variable.

5. The method of claim 4, further comprising applying the linear relationship with slope m, and intercept b, to said dependent set of controls, thereby producing a transformed set of controls that, when plotted against the independent control set, results in a straight line that has a slope equal to one and an intercept equal to zero.

6. The method of claim 4, wherein said applying comprises producing a normalized set of assay data from said assay data for said first population, wherein a value, y' in said normalized set is obtained from a value, y, in said first population of assay data by the formula $y' = (y-b)/m$.

7. The method of any one of claims 1- 6, wherein said assay data comprises microarray data.

8. The method of claim 7, wherein said first set of controls is disposed on a first microarray and said second set of controls is disposed on a second microarray, or wherein said first set of controls and said second set of controls are both disposed on the same microarray, or wherein said first set of controls comprises a first plurality of features, and said second set of controls comprises a second plurality of features such that each feature in said first plurality of features is subject to equivalent experimental conditions to a replicate feature in said second plurality of features.

9. The method of any one of claims 1- 8, wherein said assay data comprises measurements of intensity of light scattered from light scattering particles, or wherein said assay data comprises measurements of intensity of light emitted by fluorescent labels.

10. A method of ratiometric analysis, performed on assay data that comprises an array of features, said method comprising:

selecting a first population of assay data and a second population of assay data, wherein said first population comprises a dependent set of controls and
said second population comprises an independent set of controls;
obtaining a linear relationship between said independent set of controls and
said dependent set of controls; and
applying said linear relationship to said first population of assay data, thereby producing a first normalized assay data;
obtaining a second linear relationship between said second set of controls and
said first set of controls, wherein said first set of controls is treated as an independent variable, and said second set of controls is treated as a dependent variable in said linear relationship;
applying said second linear relationship to said second population of assay data, thereby producing a second normalized assay data;
calculating a ratio of a value of said feature in said normalized assay data to a value of said feature in said second normalized assay data; and
identifying said feature as regulated if said ratio exceeds a threshold value.

11. The method of claim 10, additionally comprising repeating said calculating for one or more other features.

12. The method of claim 10 or 11, wherein said threshold value is 2: 1.

13. A method of identifying at least one anomalous feature in assay data, wherein said assay data comprises an array of features, said method comprising:

dividing the assay data into a first population and a second population, wherein said first population comprises a first set of controls and said second population comprises a second set of controls;

obtaining a first linear relationship between said first set of controls and said second set of controls, including setting one set of controls as a dependent variable and the other set of controls as an independent variable;

obtaining a second linear relationship between said second set of controls and said first set of controls, wherein the control set that is treated as the dependent variable in obtaining said first linear relationship is treated as an independent variable in obtaining said second linear relationship; applying said first linear relationship to said first population, thereby producing a first normalized assay data;

applying said second linear relationship to said second population, thereby producing a second normalized assay data; and

calculating a first ratio of a value of said feature in said first normalized assay data to a value of said feature in said second normalized assay data; and

calculating a second ratio of a value of said feature in said second normalized assay data to a value of said feature in said first normalized assay data; and

multiplying said first ratio by a reciprocal of said second ratio to produce a product;

identifying said feature as anomalous if said product exceeds a threshold value.

14. The method of claim 13 , wherein said assay data comprises gene expression data and said anomalous feature indicates a difference in level of gene expression between first and second data, or wherein said assay data comes from a microarray, and said anomalous feature derives from a defect or artifact in said microarray.

15. A method for comparing a first set of assay data to a second set of assay data, comprising:

identifying a first set of controls in said first set of assay data and a second set of controls in said second set of data, wherein said first set of controls and said second set of controls are treated a equivalent;

obtaining a linear relationship between said first set of controls and said second set of controls;

applying said linear relationship to said first set of assay data, thereby transforming said first set of assay data into a third frame of reference;

applying said linear relationship to said second set of assay data, thereby transforming said second set of assay data into said third frame of reference; and

within said third frame of reference, comparing a feature from said first set of assay data that is not in said first set of controls, to a feature from said second set of assay data that is not in said second set of controls.

*FIG. 1*

FIG. 2

FIG. 2A

FIG. 3

*128*  *133*  *134*

*132*

*129*  *135*

*131*

*130*

*122*

**FIG. 4B**

*131*

*135*

*132*

*135*  *136*

*136*

**FIG. 4C**

*128*

*133*  *122*

*134*

*137*

**FIG. 4A**

EP 2 309 253 A2

*FIG. 5*

EP 2 309 253 A2

*FIG. 6*

FIG. 7

FIG. 8

EP 2 309 253 A2

*FIG. 9*

*FIG. 10*

EP 2 309 253 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 31754301 P
- US 60364962 B
- US 60376049 B
- US 6214560 B, Yguerabide
- US 9706584 W
- WO 9740181 A
- US 9823160 W
- WO 9920789 A
- US 6180415 B, Schultz
- US 740615 A, Schultz
- US 317543 P
- US 60376049 P
- US 6395558 B
- US 5599668 B

**Non-patent literature cited in the description**

- **YGUERABIDE ; YGUERABIDE.** *Anal. Biochem.,* 1998, vol. 261, 157-176
- *Anal. Biochem.,* 1998, vol. 262, 137-156
- **SCHULTZ et al.** *Proc. Natl. Acad. Sci.,* 2000, vol. 97, 996-11
- **SCHUCHHARDT, J. ; BEULE, D. ; MALIK, A. et al.** Normalization Strategies for cDNA Microarrays. *Nucl. Acids Res.,* 2000, vol. 28, e47